## Europäisches Patentamt

## European Patent Office

## Office européen des brevets

(11) Veröffentlichungsnummer: **0 004 365**
**A1**

(12) **EUROPÄISCHE PATENTANMELDUNG**

(21) Anmeldenummer: 79100828.7

(51) Int. Cl.²: **A 61 M 5/20**

(22) Anmeldetag: 19.03.79

(30) Priorität: 23.03.78 DE 2812729

(71) Anmelder: **A. Nattermann & Cie. GmbH, Nattermannallee 1, D-5000 Köln 30 (DE)**

(43) Veröffentlichungstag der Anmeldung: 03.10.79 Patentblatt 79/20

(72) Erfinder: **Becker, Michael, Brucknerstrasse 6, D-5020 Frechen-Grefrath (DE)**

(74) Vertreter: **Schönwald, Karl, Dr.-Ing. et al, Deichmannhaus, D-5000 Köln 1 (DE)**

(84) Benannte Vertragsstaaten: **BE FR GB IT NL**

(54) **Injektionsvorrichtung zur intramuskulären Einspritzung, vornehmlich von Insulin.**

(57) Eine Injektionsvorrichtung zur intramuskulären Einspritzung eines Medikamentes, vornehmlich von Insulin, wird derart ausgestattet, dass bei einhändiger Bedienung nach einem schnellen Einstechvorgang das Einspritzen der Flüssigkeit wahlweise mit unterschiedlicher Geschwindigkeit durchgeführt werden kann. Bei der Injektionsvorrichtung (1), bei der eine die Spritze (4) aufnehmende Halterung (9) gegen die Wirkung einer Feder spannbar und in der zurückgezogenen Stellung mittels einer Rastklinke (17) arretierbar ist, ist eine Handbetätigungsvorrichtung (3) für das Vordrücken des Kolbens (6) und der Kolbenstange (7, 7a) der Spritze (4) angeordnet, mit der einerseits auf die Rastklinke (17) und anderseits auf die Kolbenstange (7, 7a) der Spritze (4) eingewirkt wird. Es kann eine verschiebbar gelagerte Schubstange (23) vorgesehen sein, deren vorderes Ende (23a) mittelbar auf die Rastklinke (17) wirkt und deren hinteres Ende (23b) unmittelbar mit dem Ende der Kolbenstange (7) der Spritze (4) zusammengreift.

Injektionsvorrichtung zur intramuskulären Einspritzung, vornehmlich von Insulin

Die Erfindung bezieht sich auf eine Injektionsvorrichtung zur intramuskulären Einspritzung, vornehmlich von Insulin u.dgl., mit einer Spritze und einem die Spritze aufnehmenden Gehäuse, in dem ein Betätigungsmechanismus für den Einstechvorgang der Kanüle der Spritze und für das Vordrücken der Kolbenstange und des Kolbens der Spritze vorgesehen ist.

Es sind Injektionsvorrichtungen der vorstehend genannten Art bekannt, die eine die Spritze aufnehmende zylinderförmige Halterung aufweisen. Hierbei ist die Halterung gegen die Wirkung einer Feder spannbar und in der zurückgezogenen Stellung mittels einer Rastklinke arretierbar vorgesehen. Die Handhabung einer solchen Injektionsvorrichtung kann in der Weise vorgenommen werden, daß nach Ansetzen der Injektionsvorrichtung

Telefon: (02 21) 23 45 41 - 4 · Telex: 888 2307 dopa d · Telegramm: Dompatent Köln

der Rasthebel mit einem Finger einer Hand lösbar ist, die zugleich die Vorrichtung hält, während mit einem Finger derselben Hand der Kolben der Spritze vorgedrückt werden kann.

Die DE-AS 15 66 606 beschreibt eine selbsttätige Injektionsvorrichtung mit einem durch eine Feder in Injektionsrichtung bewegbaren Kolben und einem am Kolben befestigten Druckstößel zum Auspressen der Injektionsflüssigkeit. Diese Vorrichtung ist nur für Ampullen geeignet. Außerdem wird das Einspritzen der Injektionsflüssigkeit durch eine Feder vorgenommen, die sich in einem mit einer technischen Hilfsflüssigkeit gefüllten Zylinder befindet. Eine Regulierung der Geschwindigkeit wird durch Drosselung der hydraulischen Hilfsflüssigkeit in einem Umlauf vorgenommen.

Aus der Gebrauchsmusterschrift 76 38 511 ist ein Insulin-Injektions-Automat bekannt, bei dem das Injektionsgerät mit einer vor der Injektion zu spannenden Schraubenfeder und mit einem gegen den Federdruck verriegelbaren Rastkolben versehen ist, der in einer die Feder enthaltenden zylindrischen Federhülse längsverschiebbar geführt ist. Ein solches Injektionsgerät ist zwar einhändig bedienbar; sowohl das Einschießen der Kanüle in den Körper als auch das Injizieren des Medikaments vom Injektionsgerät werden jedoch automatisch in einem einzigen Bewegungsablauf vorgenommen. Bei einer solchen Automatik kann das Einspritzen des Medikaments von Hand nicht unterbrochen werden.

Aufgabe der Erfindung ist es, eine Injektionsvorrichtung zur intramuskulären Einspritzung, vornehmlich von Insulin, mit einer Spritze vorzusehen, mit der bei einhändiger Bedienung nach einem schnellen Einstechvorgang der Kanüle das Einspritzen des Medikaments wahlweise schnell oder langsam, auch unter Einschaltung von Haltemomenten, durchgeführt werden kann. Die Injektionsvorrichtung der anfangs genannten Art, bei der eine zylinderförmige, die Spritze aufnehmende Halterung gegen die Wirkung einer Feder spannbar und in der zurückgezogenen Stellung mittels einer Rastklinke arretierbar ist, zeichnet sich gemäß der Erfindung dadurch aus, daß für das Vordrücken des Kolbens und der Kolbenstange der Spritze eine Hand-Betätigungsvorrichtung in dem Gehäuse angeordnet ist, mit der einerseits auf die Rastklinke und andererseits auf die Kolbenstange der Spritze eingewirkt werden kann.

Durch eine solche Ausbildung der Injektionsvorrichtung wird erreicht, daß durch die einhändige Handhabung des Injektionsgerätes eine Vereinigung von Vorzügen erzielt wird, die gerade bei dem Spritzen von Insulin von außerordentlicher Wichtigkeit ist. Mit ein und derselben Betätigungsvorrichtung wird mit derselben Hand einerseits ein schneller Einstechvorgang der Injektionsnadel veranlaßt, andererseits wird das Verstellen des Kolbens der Spritze zum Eintreiben des Medikaments in den Muskel vorgenommen, wobei ein Umsetzen der Hand oder des Bedienungsfingers nicht erforderlich ist. Das Injizieren kann rein gefühlsmäßig durchgeführt werden, was besonders für Diabetiker wichtig ist, da Insulin langsam injiziert werden muß. Das Injektionsgerät ist handlich, und die Injektion kann in einfacher und sicherer Weise auch an jeder schwer zugänglichen Stelle des menschlichen Körpers vorgenommen werden.

Gemäß einem weiteren Merkmal der Erfindung kann zur Vereinigung eines schnellen Einstechvorganges für die Injektionsnadel und eines langsamen Vordrückens des Mediums aus der Spritze in dem Gehäuse des Gerätes eine Schubstange verschiebbar gelagert sein, deren vorderes Ende mittelbar auf die Rastklinke einwirkt und deren hinteres Ende mit dem Ende der Kolbenstange der Spritze zusammengreift. Die Schubstange kann mit einem zu dem Gehäuse verschiebbaren Gestänge verbunden sein. Dieses ist vorteilhaft als Zahngestänge mit einem zwischen den Zahnstangen angeordneten Zahnrad ausgebildet, wobei eine der Zahnstangen unter Federwirkung stehen kann. Auf diese Weise wird beim Betätigen ein gutes Injektionsgefühl erreicht, da das Handgefühl durch das Zahngestänge unmittelbar auf den Kolben der Injektionsspritze übertragen wird. Statt des Zahnrades kann auch ein aus mehreren Zähnen bestehendes Über- oder Untersetzungsgetriebe verwendet werden.

Die Erfindung betrifft weiterhin die Maßnahme, daß die untere Zahnstange mit einem zu dem Gehäuse längsbewegbaren Schieber geführt ist, der vorzugsweise als Abzugshahn ausgebildet ist, wobei das Gehäuse ein pistolenartiges Griffstück aufweist. Durch eine solche Ausbildung des Gehäuses mit der Betätigungsvorrichtung ist es ermöglicht, daß die Injektionsvorrichtung sehr ruhig in der Hand gehalten werden kann und das Einstechen der Injektionsnadel sowie das Eintreiben des Medikaments bei unverändertem Griff der Hand mit einem Finger durchgeführt werden kann. Mit demselben Abzugshahn wird die Injektionsnadel in die Haut geschossen, und es erfolgt beim weiteren Durchziehen des Abzugshahnes das Injizieren des Medikaments von Hand, je nach Bedarf und Abhängigkeit von dem Handgefühl.

Die Schubstange oder obere Zahnstange besitzt vorteilhaft einen Ansatz, an dem eine Einstellvorrichtung in Form einer verstellbaren Gewindespindel angebracht ist, so daß man den Beginn des Einspritzvorganges wählen kann.

Die zylinderförmige Halterung ist vorteilhaft in einer mit einem Kopfstück versehenen Hülse gelagert, wobei die Halterung unmittelbar mit dem Gehäuse durch eine Zugfeder verbunden ist. Das verstellbare Kopfstück dient zum Schutz der vorstehenden Injektionsnadel und zugleich auch einer weiteren Hülse, in der sich ein Vorratsbehälter mit dem Medikament befinden kann. Durch diese weitere bzw. Zusatzhülse kann das Aufziehen des Medikaments in die Spritze leicht durchgeführt werden.

Die Erfindung wird anhand eines in der Zeichnung dargestellten Ausführungsbeispiels nachstehend erläutert.

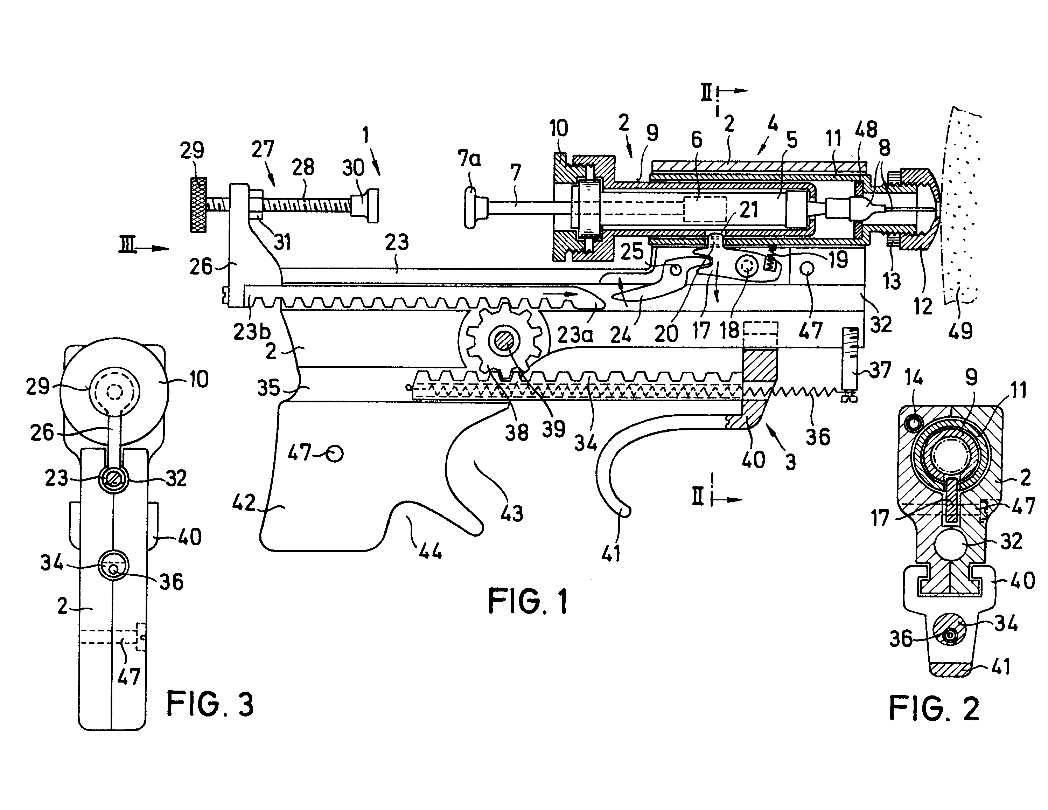

Fig. 1 zeigt ein Ausführungsbeispiel der Injektionsvorrichtung zur intramuskulären Einspritzung, vornehmlich von Insulin, im Aufriß und im Längsschnitt, schematisch.

Fig. 2 ist ein Querschnitt nach der Linie II-II der Fig. 1.

Fig. 3 stellt eine Stirnansicht in Richtung des Pfeiles III dar.

Fig. 4, 5 und 6 sind Draufsichten auf die Injektionsvorrichtung der Fig. 1 und zeigen verschiedene Phasen bei der Betätigung der Injektionsvorrichtung, im Schema.

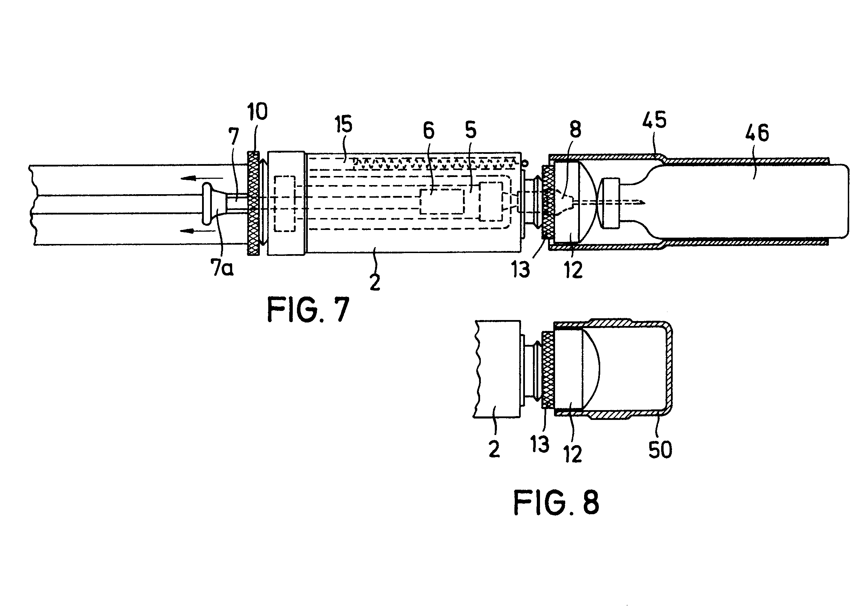

Fig. 7 und 8 zeigen auf das Kopfstück aufsteckbare Hülsen verschiedener Art zum Verschließen und zur Aufnahme eines Vorratsbehälters für das Aufziehen der Spritze.

Die Injektionsvorrichtung 1 zur intramuskulären Einspritzung, vornehmlich von Insulin, weist ein Gehäuse 2 auf, in dem eine Vorrichtung zum schnellen Einstechen der Injektionsnadel und eine Handbetätigungsvorrichtung 3 zum Injizieren des Medikaments vereinigt sind. Die Spritze 4, die aus dem Spritzenbehälter 5, einem Kolben 6, einer Kolbenstange 7 mit Knauf 7a und einer auswechselbaren Injektionsnadel 8 zusammengesetzt ist, ist in einer zylinderförmigen Halterung 9 angeordnet, in der die Spritze mittels der Gewindebuchse 10 eingespannt gehalten wird. Die Halterung 9 ist in einer Hülse 11 verschiebbar gelagert, die am vorderen Ende ein aufschraubbares Kopfstück 12 mit einer Kontermutter 13 aufweist. Das Kopfstück 12 dient zum Schutz der Injektionsnadel 8. Die Halterung 9 ist gegen die Wirkung einer Federung 14 spannbar, die in dem Gehäuse 2 angeordnet ist, wobei das eine Widerlager ein mit der Halterung 9 fest verbundener Stift 15 ist, der die Halterung zugleich gegen Verdrehen sichert, und das andere Widerlager 16 sich an dem Gehäuse 2 abstützt. Zum Sichern der die Spritze 4 aufnehmenden Halterung 9 in der zurückgezogenen Stellung dient eine Rastkline 17, die bei 18 in dem Gehäuse schwenkbar gelagert ist und unter der Wirkung einer Feder 19 steht. Die Klinke 17 weist einen Nocken 20 auf, der in einer Ausnehmung 21 der Halterung 9 eingreift.

In dem Gehäuse 2 ist eine Schubstange 23 verschiebbar gelagert, deren vorderes Ende 23a mittelbar über einen Zwischenhebel 24, der bei 25 drehbar gelagert ist, mit der Rastklinke 17 zusammengreift, während das hintere Ende 23b einen Ansatz 26 trägt, an dem eine Verstellvorrichtung 27 angebracht ist. Diese besteht aus einer Gewindespindel 28 mit einer Handhabe 29 und einer Kappe 30, wobei die Gewindespindel 28 durch eine Kontermutter 31 festgesetzt werden kann. Bei Verschieben der Schubstange 23, die durch eine Nut 32 geführt sein kann, wirkt das vordere Ende 23a mittelbar auf die Rastklinke zum Ausrasten aus der Halterung 9 und mit dem hinteren Ende 23a und den Teilen 26 und 27 auf die Kolbenstange 7, 7a der Spritze 4.

Die Handbetätigungsvorrichtung 3 weist vorteilhaft ein Gestänge auf, das als Zahngestänge ausgebildet ist. Hierbei bildet die Schubstange 23 die eine Zahnstange, während eine weitere Zahnstange 34 in der Nut 35 des Gehäuses geführt ist und mittels der Feder 36 unter Zugspannung gehalten werden kann. Die Feder 36 ist einerseits an dem Widerlager 37 und andererseits an dem hinteren Ende der Zahnstange 34 befestigt. Zwischen den beiden Zahnstangen 23 und 34 befindet sich ein kleines Zahnrad 38, das bei 39 in dem Gehäuse 2 drehbar gelagert ist. Statt eines Zahnrades 38 kann auch ein aus mehreren Zahnrädern bestehendes Getriebe zur Übersetzung oder Untersetzung verwendet werden.

Die Zahnstange 34 ist mit einem Schieber 40 fest verbunden, der an dem Gehäuse 2 längsverschiebbar gelagert ist. Der Schieber 40 ist in Form eines Abzugshahnes 41 ausgebildet, wobei das Gehäuse 2 mit einem pistolenartigen Griffstück 42 versehen ist. Die Ausnehmung 43 an dem Griffstück 42 dient zur Aufnahme des Abzugshahnes 41, während die Aussparung 44 vorteilhaft zur Aufnahme des Mittelfingers der den pistolenartigen Griff 42 umschließenden Hand dient.

Auf das Kopfstück 12 ist eine Zusatzhülse 45 aufsteckbar, die zur Aufnahme eines Vorratsbehälters 46 für das Medikament dient. Dadurch ist die Gewähr gegeben, daß der Vorratsbehälter 46 zentrisch zur Injektionsnadel 8 angesetzt werden kann, wodurch beim Aufziehen des Medikaments mittels der Spritze 4 ein Verbiegen der Injektionsnadel 8 verhindert wird. Bei Nichtgebrauch der Injektionsvorrichtung kann auf das Kopfstück 12 eine Schutzhülse 50 aufgesteckt werden, um den vorderen Teil der Spritze vor Eindringen von Schmutz und sonstigen Verunreinigungen zu schützen.

Das Gehäuse 2 ist vorteilhaft aus zwei Teilen zusammengesetzt, die durch Schrauben 47 und auch den Lagerstift 39 fest miteinander verbunden gehalten werden.

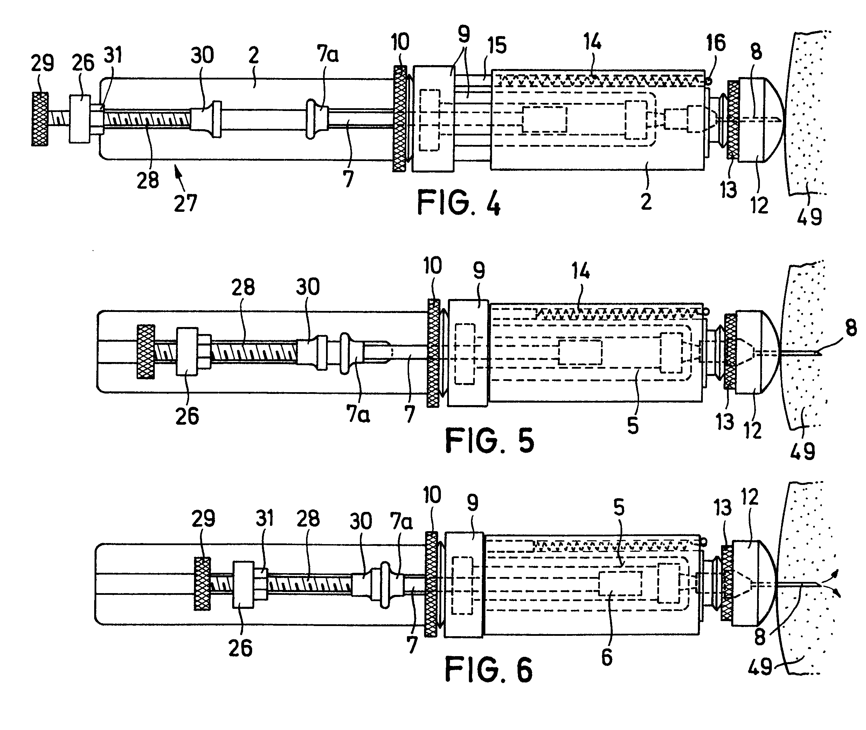

Zum Injizieren wird die gefüllte Spritze 4 zusammen mit der Halterung 9 zurückgezogen, bis der Nocken 20 in die Ausnehmung 21 der Halterung 9 einfallen kann, wobei die Federung 14 gespannt wird. Bei Betätigung des Abzugshahnes 41, d.h. bei Zurückziehen des Schiebers 40 zusammen mit der unteren Zahnstange 34 mit dem Zeigefinger, wird die Schubstange 23 über das Zahnrad 38 in Richtung des Pfeiles nach vorn gestoßen. Das vordere Ende 23a unterfängt den Hebel 24, durch dessen Schwenkung in Pfeilrichtung die Nocke 20 aus der Aussparung 21 der Halterung 9 gebracht wird. Unter der Wirkung der Federung 14 schnellt die Halterung 9 zusammen mit der Spritze 4 vor, wodurch die Injektionsnadel 8 in die Muskulatur 49 der betreffenden Person geschossen wird. Beim weiteren Durchziehen des Abzugshahnes 41 bis zu der Ausnehmung 43 des Griffstückes 42 wird das in der Spritze 4 befindliche Medikament langsam nach Handgefühl injiziert, wobei die Vorrichtung 27 bis zu dem Knauf 7a der Kolbenstange 7 der Spritze 4 herangeführt wird. Fig. 4 zeigt die Stellung der Teile vor dem Einschießen der Injektionsnadel in den Körperteil, während Fig. 5 eine Stellung veranschaulicht, bei der nach eingeschossener Injektionsnadel 8 die entsprechend eingestellte Vorrichtung 27 dicht vor dem Knauf 7a der Kolbenstange 7 der Spritze 4 liegt. Bei weiterem langsamem Durchziehen des Abzugshahnes 41 stößt das Teil 30 an den Knauf 7a der Kolbenstange 7 an und drückt den Kolben 6 in der Spritze 4 nach vorn, welcher Zustand in der Fig. 6 dargestellt ist. Das Auslösen des Einstechvorganges und das nachfolgende Eindrücken des Medikaments aus der Spritze 4 mittels der Handbetätigungsvorrichtung 3 kann in einer stetigen Durchziehbewegung an dem Abzugshahn 41 erfolgen, wobei je nach der in der Spritze 4 aufgezogenen Menge des Medikamentes, d.h. je nach der Stellung des Kolbens 6 mit der Kolbenstange 7, nach dem Einstechvorgang das Ausdrücken des Medikaments aus der Spritze 4 vor sich gehen kann. Bei dem Einschießen der Injektionsnadel 8 wird die Halterung 9 und damit auch die Nadel kurz vor dem Ende des Einstiches durch einen in der Hülse 11 befindlichen Gummipuffer 48 abgedämpft. Dadurch wird ein psychisch störendes Schußgeräusch und auch ein eventuelles Abspringen der Injektionsnadel vom Haltekonus verhindert.

Die Einstichtiefe der Injektionsnadel 8 ist durch die Verstellbarkeit des Kopfstückes 12 mit der Kontermutter 13 einstellbar. Zugleich wird die Injektionsnadel durch das Kopfstück 12 geschützt, welches auch zur Aufnahme einer Zusatzhülse 45 für das neue Aufziehen des Medikaments in die Spritze aus der Vorratsflasche 46 oder einer Schutzkappe 50 dient.

Die Injektionsvorrichtung kann für jedes Medikament verwendet werden, welches mittels einer Spritze zu injizieren ist.

Bei der Injektionsvorrichtung kann man mit einem Finger der die Vorrichtung haltenden Hand die Injektionsnadel einschießen und das Serum injizieren, wobei durch die pistolenartige Form eine ruhige Handhabung erreicht wird und man die Injizierung auch an schwer zugänglichen Körperstellen vornehmen kann. Ferner kann man einerseits bei der Injektionsvorrichtung die Nadelstichtiefe einstellen und andererseits mit einem Finger derselben Hand die Injektion beliebig unterbrechen oder nach Handgefühl zu Ende führen, was für Diabetiker wichtig ist.

Im Zusammenhang mit der Zusatzhülse zum Aufziehen des Serums kann auch eine einarmige Person die Injektionsvorrichtung gebrauchsfertig machen, nämlich folgende Handhabungen mit einer Hand vornehmen: Aufziehen des Serums, Spannen der Spritze und Injizieren. Die Zusatzhülse dient ferner zum Aufbewahren der Vorratsflasche für das Serum und schützt sie gegen Zerbrechen, was im Zusammenhang mit einem Tragebesteck auf Reisen wichtig ist. Die Injektionsvorrichtung ist so gestaltet, daß sie in einem kleinen Täschchen überall mitgeführt werden kann.

A n s p r ü c h e

1. Injektionsvorrichtung zur intramuskulären Einspritzung eines Medikaments, vornehmlich von Insulin, mit einer Spritze und einem die Spritze aufnehmenden Gehäuse, in dem ein Betätigungsmechanismus für den Einstechvorgang der Injektionsnadel der Spritze und für das Vordrükken der Kolbenstange und des Kolbens der Spritze vorgesehen ist, wobei eine zylinderförmige, die Spritze aufnehmende Halterung gegen die Wirkung einer Feder spannbar und in der zurückgezogenen Stellung mittels einer Rastklinke arretierbar ist, dadurch gekennzeichnet, daß für das Vordrücken des Kolbens (6) und der Kolbenstange (7,7a) der Spritze (4) eine Handbetätigungsvorrichtung (3) in dem Gehäuse (2) angeordnet ist, mit der einerseits auf die Rastklinke (17) und andererseits auf die Kolbenstange (7,7a) der Spritze (4) einwirkbar ist.

2. Injektionsvorrichtung nach Anspruch 1, dadurch gekennzeichnet, daß in dem Gehäuse (2) eine Schubstange (23) verschiebbar gelagert ist, deren vorderes Ende (23a) mittelbar auf die Rastklinke (17) wirkt und deren hinteres Ende (23b) unmittelbar mit dem Ende der Kolbenstange (7) der Spritze (4) zusammengreift.

3. Injektionsvorrichtung nach Anspruch 1 oder 2, dadurch gekennzeichnet, daß die Schubstange (23) mit einem zu dem Gehäuse (2) verschiebbaren Gestänge in Verbindung steht.

4. Injektionsvorrichtung nach einem der Ansprüche 1 bis 3, dadurch gekennzeichnet, daß das Gestänge als Zahngestänge mit einem zwischen den Zahnstangen (23,34) angeordneten Zahnrad (38) ausgebildet ist, und daß eine der Zahnstangen unter Federwirkung (36) steht.

5. Injektionsvorrichtung nach einem der Ansprüche 1 bis 4, dadurch gekennzeichnet, daß die untere Zahnstange (34) mit einem zu dem Gehäuse (2) längsbewegbaren Schieber (40) verbunden ist, der einen Abzugshahn (41) aufweist, wobei das Gehäuse (3) ein pistolenartiges Griffstück (42) besitzt.

6. Injektionsvorrichtung nach einem der Ansprüche 1 bis 5, dadurch gekennzeichnet, daß die obere Zahnstange (23) einen Ansatz (26) aufweist, an dem eine Einstellvorrichtung (27) in Form einer verstellbaren Gewindespindel (28-31) angebracht ist.

7. Injektionsvorrichtung nach einem der Ansprüche 1 bis 6, dadurch gekennzeichnet, daß zwischen der Rastklinke (17) und der oberen Zahnstange (23) ein drehbar gelagerter Zwischenhebel (24) angeordnet ist.

8. Injektionsvorrichtung nach einem der Ansprüche 1 bis 7, dadurch gekennzeichnet, daß die zylinderförmige Halterung (9) in einer mit einem Kopfstück (12) versehenen Hülse (11) gelagert und unmittelbar mit dem Gehäuse (2) durch eine Zugfeder (14) verbunden ist.

9. Injektionsvorrichtung nach einem der Ansprüche 1 bis 8, dadurch gekennzeichnet, daß das Kopfstück (12) auf einen Gewindeansatz der Hülse (11) aufschraubbar ist, und daß
auf das Kopfstück (12) eine weitere, einen Vorratsbehälter (46) aufnehmende Zusatzhülse (45) oder eine Verschlußkappe (50) aufsteckbar ist.

FIG. 1

FIG. 3

FIG. 2

-1/3-

0004365

FIG. 4

FIG. 5

FIG. 6

- 2/3 -

0004365

FIG. 7

FIG. 8

0004365

# EUROPÄISCHER RECHERCHENBERICHT

Europäisches Patentamt

0004365
Nummer der Anmeldung

EP 79 100 828.7

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | <u>US - A - 4 067 334</u> ( J.G. HALLER)<br>* Spalte 4, Zeilen 40 bis 49; Spalte 5, Zeilen 3 bis 11; Ansprüche 1,2, 7 bis 12; Fig. 1 bis 4, 6,9,10, Positionen 50,102,104,78,76,34 10, 32, 70, 72, 74 *<br>-- | 1,2,3, 6,8 |
| | <u>GB - A - 1 242 060</u> (ARMOUR PHARMA-CEUTICAL)<br>* Fig. 1,2,5, Positionen 12, 16, 28 bis 34 *<br>-- | 3,4,5, 8 |
| | <u>US - A 4 026 288</u> (R. COSTA et al.)<br>* Ansprüche 1 und 2; Fig. 1,2, Positionen 32,33,52,56,63,50,61 *<br>-- | 4,5 |
| | <u>DE - U - 7 204 481</u> (F.H. ADAMASZEK)<br>* Fig. 1 Positionen 3,4; Seite 5, Zeile 9 *<br>-- | 9 |
| D,A | <u>DE - B - 1 566 606</u> (S. GRANT)<br>* gesamtes Dokument *<br>-- | |
| D,A | <u>DE - U - 7 638 511</u> (W. HASELMEIER)<br>* gesamtes Dokument *<br>---- | |

### KLASSIFIKATION DER ANMELDUNG (Int.Cl.²)

A 61 M 5/20

### RECHERCHIERTE SACHGEBIETE (Int. Cl.²)

A 61 M 5/20

### KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 20-06-1979 | DROPMANN |

EPA form 1503.1 06.78